(19) Europäisches Patentamt
European Patent Office
Office européen des brevets

(11) Publication number: 0 127 471 B1

(12) **EUROPEAN PATENT SPECIFICATION**

(45) Date of publication of patent specification: **09.09.87**

(51) Int. Cl.$^4$: **C 07 F 9/12,** A 61 K 31/665, A 61 K 7/48

(21) Application number: **84303566.8**

(22) Date of filing: **25.05.84**

(54) **Phosporic acid diesters, their salts, compositions containing them and a process for producing said diesters and salts.**

(30) Priority: **30.05.83 JP 96428/83**

(43) Date of publication of application: **05.12.84 Bulletin 84/49**

(45) Publication of the grant of the patent: **09.09.87 Bulletin 87/37**

(84) Designated Contracting States: **DE FR GB IT**

(56) References cited:
**FR-A-1 489 249**

**CHEMICAL ABSTRACTS, vol. 96, no. 21, 24th May 1982, page 594, no. 179855q, Columbus, Ohio, US; F. KUZUDANI: "Effect of dl-alpha-tocopherol phosphoric acid ester on platelets"**

**CHEMICAL ABSTRACTS, vol. 73, no. 13, 28th September 1970, page 331, no. 66429k, Columbus, Ohio, US**

**CHEMICAL ABSTRACTS, vol. 101, no. 6, 6th August 1984, page 324, no. 43589v, Columbus, Ohio, US**

(73) Proprietor: **Senju Seiyaku Kabushiki Kaisha also known as Senju Pharmaceutical Co. Ltd.**
**6-1, Hiranomachi 3-chome**
**Higashi-ku Osaka (JP)**

(72) Inventor: **Ogata, Kazumi**
**8B-701, Kamishinden 4-chome**
**Toyonaka Osaka 565 (JP)**

(74) Representative: **Burford, Anthony Frederick et al**
**W.H. Beck, Greener & Co. 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel phosphoric acid diesters and their salts and a process for producing the same.

The phosphoric acid diesters of the present invention have a structure in which two out of the three hydroxyl groups of phosphoric acid are esterified with one hydroxyl group each of α-tocopherol and ascorbic acid, respectively.

Ascorbic acid is an antiscurvy agent and suppresses the deposit of melanin to which liver-spot and ephelis are attributable. Moreover, it has been recently reported to have an anti-cancer effect.

On the other hand, α-tocopherol does not exhibit directly anti-cancer action, but it is reported that people having a daily intake of α-tocopherol are less susceptible to cancer. In addition, α-tocopherol is regarded as effective against climacteric hazards such as numbness of hands and feet, and, like ascorbic acid, is also concerned in oxidation-reduction in the living body, which has suggested in recent years that it is effective for treatment of cataracts.

Compounds having ascorbic acid and α-tocopherol linked through phosphoric acid are not yet known. The present inventor, after repeated intensive research, succeeded in the synthesis of the compounds of the present invention, which comprise 1 mole each of ascorbic acid and α-tocopherol linked to 1 mole of phosphoric acid in the diester form.

The compounds of the present invention are considered to have the structure as represented by the following Formula I.

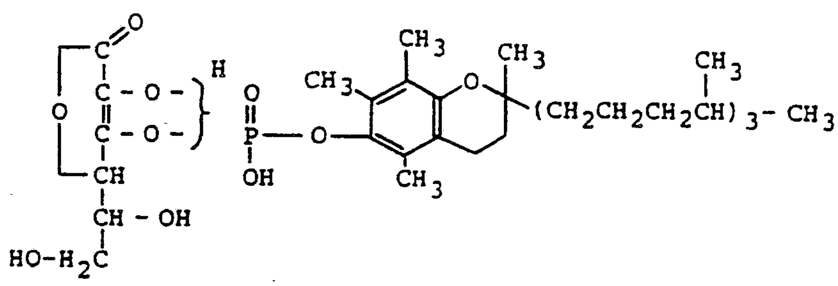

The present invention is concerned with phosphoric acid diesters of the Formula I and their salts, and with a process for producing phosphoric acid diesters of the Formula I and their salts. The process comprises reacting α-tocopherol with a halogenophosphorylating agent, reacting the resultant product with ascorbic acid having the hydroxyl groups in the 5- 6-positions protected with protecting groups, and removing the said protecting groups.

In the process according to the present invention, a halogenophosphorylating agent is reacted with α-tocopherol. Preferred examples of the halogenophosphorylating agent include phosphorus oxytrichloride and phosphorus oxytribromide. The reaction proceeds readily in such a non-reactive solvent as benzene in the presence of a acid binding agent, for example, pyridine. When phosphorus oxytrichloride is employed as the halogenophosphorylating agent, the reaction sequence is illustrated in the following manner:—

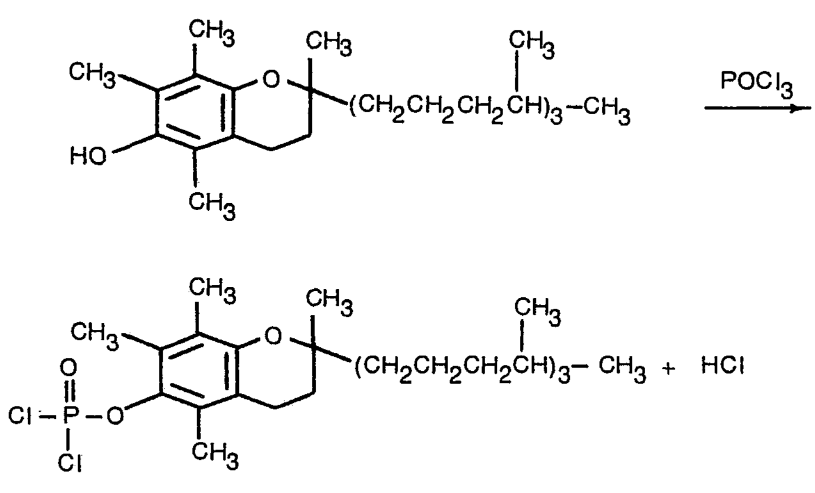


II

The product of Formula II above is reacted with ascorbic acid having the hydroxyl groups in the 5- and 6-positions protected with protecting groups. The protecting groups can be selected from the various protecting groups known in the field of synthesis of ascorbic acid. Suitably, the isopropylidene group can

be employed. The reaction proceeds in a solvent such as tetrahydrofuran in the presence of an acid binding agent such as pyridine. The protecting groups are eliminated from the reaction product. This elimination usually is carried out by hydrolysis under mild conditions, for example, by us of 1N hydrochloric acid. Even when a halogenophosphoric acid group is present in the above-mentioned reaction product, it can be converted into a phosphoric acid group by replacing its halogens by hydroxyl groups simultaneously with the removal of the protecting groups in the above-mentioned hydrolysis.

The compounds of the present invention are more crystalline in the form of salts than as free acids. Their alkali metal salts, such as sodium and potassium salts, are highly soluble in water, whereas their alkaline earth metal salts, such as calcium salts, are insoluble. Therefore, the type of salt can be selected according to the required purpose.

In order to convert the free acids into alkali metal salts, in general, it is preferable to neutralize with alkali metal hydroxides.

In the drawings, Figures 1 and 2 show respectively the infrared adsorption spectra of the potassium salt and the free acid of the compound of the present invention, having a DL-α-tocopherol residue.

The compounds of the present invention, either in the form of free acid or suitable salt, can be processed into various pharmaceutical preparations, such as injections, collyria, tablets and capsules, by known procedures. The compounds of the present invention do not have a reducing action and are therefore stable in air. However, a reducing action is readily restored, for example, when they are heated with hydrochloric acid. Consequently, they can be administered to the living body as stable pharmaceutical preparations, and at the same time, can be expected to exhibit the actions of Vitamins C and E in the body through splitting off of the phosphoric acid ester groups by the action of phosphatase, etc.

Also, the compounds of the present invention, in the form of salts such as sodium and potassium salts, exhibit enhanced solubility in water, and are free from the disadvantage of turbidity at about neutral pH and precipitation on addition of sodium chloride, which disadvantage occurs with the phosphoric acid ester of α-tocopherol.

The compounds of the present invention can, for example, be used as prophylactic, therapeutic or diagnostic agents for cataracts and climacteric hazards, and also as ingredients for cosmetics having skin-beautifying action.

The following Examples illustrate the present invention.

## Example 1

L-ascorbic acid, DL-α-tocopherol phosphoric acid ester potassium salt

In 50 ml of benzene 6.12 g of phosphorus oxytrichloride is dissolved, and a mixture of 8.6 g (0.02 mole) of DLα-tocopherol and 9.5 g of pyridine dissolved in 50 ml of benzene is added dropwise to the solution under stirring. After the dropwise addition is completed, stirring is continued for another 3 hours, and the precipitated pyridine hydrochloride is filtered off. The filtrate is evaporated under reduced pressure, and 30 ml of benzene is added to the resultant residual, oily substance.

5.2 g (0.024 mole) of 5,6-isopropylideneascorbic acid resulting from the acetalisation of L-ascorbic acid and 3.2 g of pyridine are dissolved in 120 ml of tetrahydrofurane (THF), and the solution is added dropwise to the above benzene solution. After the dropwise addition is completed, stirring is continued for about 1 hour, the precipitated pyridine hydrochloride is filtered off, and the filtrate is freed from the solvent under reduced pressure. The resultant oily substance is dissolved in 30 ml of ethyl alcohol, and 150 ml of 1N hydrochloric acid is added to the solution, followed by heating under reflux for about 20 minutes. The solution is cooled, and extracted with ethyl acetate. The organic layer is dried over anhydrous sodium sulfate and the ethyl acetate is distilled off. There is obtained the crude free acid as residue.

The crude free acid is dissolved in about 100 ml of ethyl alcohol, and a solution of potassium hydroxide in ethyl alcohol is gradually added dropwise to the solution until the pH of the solution becomes neutral, whereby slightly brown-tinged white crystals separate out. The crystals are recovered by filtration and recystallized from water-ethyl alcohol-acetone to yield 7.5 g of white powdery crystals.

Melting point: Carbonization gradually begins at temperature of about 210°C.

Ultraviolet absorption spectrum (UV): It shows an absorption maximum of about 257 nm (in water).

Silica-gel thin-layer chromatography: RF = 8.1 (ethyl alcohol: acetone: water = 10:4:1).

Elemental analysis, for $C_{35}H_{55}O_{10}PK_2 \cdot H_2O$

Calcd.: C, 55.09%   H, 7.53%

Found: C, 55.32%   H, 7.65%

Infrared absorption spectrum (KBr disk): As shown in Fig. 1.

## Example 2

L-ascorbic acid, DL-α-tocopherol phosphoric acid ester sodium salt

In 30 ml of water 5 g of the L-ascorbic acid-DL-α-tocopherol phosphoric acid ester potassium salt obtained in Example 1 is dissolved and the solution is made acidic by the addition of hydrochloric acid. After extraction with ethyl acetate, the organic layer is freed of the ethyl acetate to yield the free acid, L-ascorbic acid-DL-α-tocopherol phosphoric acid ester (the absorption maximum of the UV spectrum is at 285 nm, in water). The infrared absorption spectrum is shown in Fig. 2. The free acid is dissolved in ethyl alcohol, and a 30% aqueous sodium hydroxide solution is gradually added to the solution until the solution

becomes neutral, whereby there are obtained white crystals. The crystals are recovered by filtration, washed with ethyl alcohol and dried to give about 4 g of the desired compound.

Elemental analysis, for $C_{35}H_{55}O_{10}PNa_2 \cdot H_2O$

Calcd.: C, 57.52% H, 7.86%

Found: C, 57.65% H, 7.98%

Example 3

L-ascorbic acid, DL-α-tocopherol phosphoric acid ester calcium salt.

In 100 ml of water 5 g of the potassium salt obtained in Example 1 is dissolved, and 2 g of calcium chloride is added to the solution. The precipitated white crystals are recovered by filtration, washed with water and dried to yield about 4.5 g of the desired compound.

Elemental analysis, for $C_{35}H_{55}O_{10}PCa \cdot H_2O$

Calcd.: C, 57.99% h, 7.93%

Found: C, 58.12% H, 8.22%

Example 4

L-ascorbic acid, D-α-tocopherol phosphoric acid ester potassium salt.

D-α-tocopherol acetate (produced by Amakasu Chemical Co. of Japan) is hydrolysed by heating it in a solution of 50% aqueous sulfuric acid and ethyl alcohol (ratio aobut 1:5). Using the D-α-tocopherol thus obtained, the procedure of Example 1 is repeated.

By the above procedure, there is obtained 7.5 g of slightly brown tinged white powdery crystals $[\alpha]_D^{23}$ + 40.3° (c = 1, $H_2O$).

Preparation Example 1

Injection:

| | |
|---|---|
| L-ascorbic acid, D-α-tocopherol phosphoric acid ester potassium salt | 0.2 g |
| Glucose | 5 g |

The above ingredients are dissolved in distilled water for injection, and the solution is adjusted to pH 6.8 with 1N hydrochloric acid and made up to 100 ml total volume with distilled water. The solution is filtered, and 2 ml of the filtrate is filled under sterile conditions into glass ampoules, which are then sealed.

Preparation Example 2

Collyria:

| | |
|---|---|
| L-ascorbic acid, DL-α-tocopherol phosphoric acid ester potassium salt | 0.5 g |
| Boric acid | 1.8 g |
| Benzalkonium chloride | 0.005 g |

The above ingredients are dissolved in sterilized, purified water, and the solution is adjusted to pH 7.3 with 1N sodium hydroxide and made up to 100 ml of the total volume.

Preparation Example 3

Tablets:

| | |
|---|---|
| L-ascorbic acid, DL-α-tocopherol phosphoric acid ester calsium salt | 100 g |
| Lactose | 80 mg |
| Starch | 17 mg |
| Magnesium stearate | 3 mg |

The above ingredients, as a raw material for one tablet, are molded into a tablet by a conventional method. The tablet may be coated with sugar, if required.

## Preparation Example 4

Syrup:

| | |
|---|---|
| L-ascorbic acid, DL-α-tocopherol phosphoric acid ester potassium salt | 2.0 g |
| 70% aqueous solution of D-sorbitol | 70 ml |
| Methyl p-hydroxybenzoate | 0.025 g |
| Butyl p-hydroxybenzoate | 0.012 g |

The above ingredients are dissolved in sterilized, purified water. The solution is adjusted to pH 6.0 with 1N hydrochloric acid, made up to 100 ml total volume and filtered into a glass bottle.

## Preparation Example 5

Lotion:

| | |
|---|---|
| L-ascorbic acid, DL-α-tocopherol phosphoric acid ester potassium salt | 1.0 g |
| Citric acid | 0.1 g |
| Glycerin | 5.0 g |
| Ethyl alcohol | 8.0 ml |
| Methyl p-hydroxybenzoate | 0.1 g |

The above ingredients are dissolved in sterilized, purified water. The solution is adjusted to pH 6.0 with 1N sodium hydroxide, made up to 100 ml total volume and filled into a glass bottle.

## Preparation Example 6

Cream:

| | |
|---|---|
| L-ascorbic acid, DL-α-tocopherol phosphoric acid ester potassium salt | 1.0 g |
| Stearic acid | 2.0 g |
| Stearyl alcohol | 7.0 g |
| Squalane | 5.0 g |
| Octyldodecanol | 6.0 g |
| Cetyl polyoxyethylene(15) ether | 3.0 g |
| Gylcerin monostearate | 2.0 g |
| Methyl p-hydroxybenzoate | 0.2 g |
| Propyl p-hydroxybenzoate | 0.1 g |
| Sterilized, purified water | 68.7 g |

Propylene glycol and L-ascorbic acid, DL-α-tocopherol phosphoric acid ester potassium salt are dissolved in the sterilized, purified water. The solution is heated to 70°C. The other ingredients are mixed with each other, melted by heating and maintained at 70°C. The above-mentioned aqueous solution is added to the melted ingredients. The admixture is emulsified homogeneously and cooled to room temperature to obtain a toiletary cream which is filled into a suitable vessel.

## Reference Experiment

If the present compound is hydrolyzed in the living body to liberate vitamin E (v. E), the residue of the compound is the phosphate of vitamin C which is, as already known, readily converted to vitamin C (v. C) by phosphatase in the body. Therefore, the conversion of the present compound to vitamins E and C in living body can be proved by detecting Vitamin E in v. E deficient animals to which the compound is administered.

For the reason mentioned above, the DL-α-tocopherol ester compound of the present invention was administered to v. E deficient rats. Increase in the concentration of v. E in blood serum as well as the haemolysis of red blood cells were observed in the rats. No difference was observed as compared with a group of rats to which v. E was administered. This indicates that the test compound is converted to v. E in the animal body showing indirectly the conversion of the residual phosphate to v. C. The data was as follows:—

Experimental animals: Male Wister rats, aged 4 weeks.

Method: Each of the test compound and vitamin E in the same moles was added to v. E-free diet (A) to make diets, (B) and (C).

Diets

(A) v. E-free diet

(B) (A) + test compound (761 mg/Kg in diet)

(C) (A) + v. E (500 mg/Kg in diet)

Respective diets, (A), (B) and (C) were fed to three groups of the rats respectively for 32 days and the blood of the each rat was collected. V. E concentration in blood plasma and haemolysis rate with dialuric acid were determined. The results are shown in Table 1.

TABLE 1
V. E concentration in blood plasma and hemolysis rate

| Diets | V. E µg/ml | Hemolysis rate (%) |
|---|---|---|
| (A) | 3.17 | 99.5 |
| (A) | 4.39 | 86.7 |
| (A) | 3.66 | 96.6 |
| (B) | 14.39 | 0. |
| (B) | 10.98 | 0. |
| (B) | 13.96 | 0. |
| (C) | 10.24 | 0. |
| (C) | 12.68 | 0. |
| (C) | 14.26 | 0. |

**Claims**

1. A phosphoric acid diester of the formula:

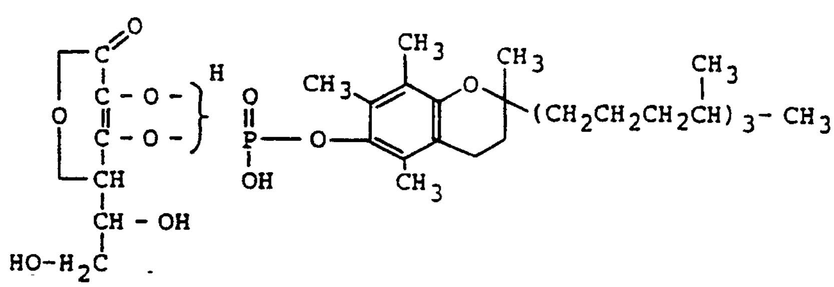

or a salt thereof.

2. A phosphoric acid diester or salt thereof as claimed in Claim 1, wherein the α-tocopherol residue is DL-α-tocopherol residue.

3. A phosphoric acid diester or a salt thereof as claimed in Claim 1, wherein the α-tocopherol residue is is D-α-tocopherol residue.

4. A phosphoric acid diester salt as claimed in any one of the preceding Claims, wherein the salt is a potassium salt.

5. A phosphoric acid diester salt as claimed in any one of Claims 1 to 3, wherein the salt is a sodium salt.

6. A phosphoric acid diester salt as claimed in any one of Claims 1 to 3, wherein the salt is a calcium salt.

7. A process for producing a phosphoric acid diester or salt thereof as claimed in any one of the preceding Claims, which comprises reacting α-tocopherol with a halogenophosphorylating agent, reacting the resultant product with ascorbic acid having the hydroxy groups in the 5- and 6-positions protected with protecting groups, and removing the protecting groups.

8. A process as claimed in Claim 7, wherein the halogenophosphorylating agent is phosphoryl oxytrichloride or oxytribromide and the protecting group is an isopropylidene group.

9. A process as claimed in Claim 7, wherein the protecting groups are removed by hydrolysis with hydrochloric acid.

10. A pharmaceutical composition which comprises a phosphoric acid diester or pharmaceutically acceptable salt thereof as claimed in any one of Claims 1 to 6 and a carrier.

11. A cosmetic composition which comprises a phosphoric acid diester or cosmetically acceptable salt thereof as claimed in any one of Claims 1 to 6 and a carrier.

12. A phosphoric acid diester or pharmaceutically acceptable salt thereof as claimed in any one of Claims 1 to 6 for use in the treatment of the human or animal body by therapy or of diagnosis practiced on the human or animal body.

13. A phosphoric acid diester or pharmaceutically acceptable salt thereof as claimed in any one of Claims 1 to 6 for use in the administration of vitamins C and E to the human or animal body.

**Patentansprüche**

1. Phosphorsäurediester der Formel:

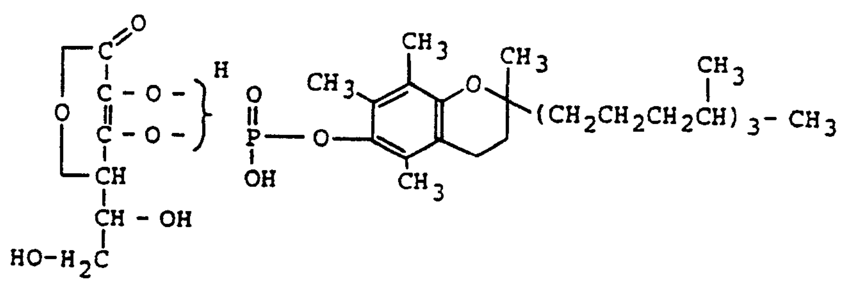

oder ein Salz davon.

2. Phosphorsäurediester oder ein Salz davon nach Anspruch 1, worin der α-Tocopherolrest ein DL-α-Tocopherolrest ist.

3. Phosphorsäurediester oder ein Salz davon nach Anspruch 1, worin der α-Tocopherolrest ein D-α-Tocopherolrest ist.

4. Phosphorsäurediestersalz nach einem der vorhergehenden Ansprüche, worin das Salz das Kaliumsalz ist.

5. Phosphorsäurediestersalz nach einem der Ansprüche 1 bis 3, worin das Salz das Natriumsalz ist.

6. Phosphorsäurediestersalz nach einem der Ansprüche 1 bis 3, worin das Salz das Calciumsalz ist.

7. Verfahren zur Herstellung eines Phosphorsäurediesters oder Salzes davon nach einem der vorhergehenden Ansprüche, wobei man α-Tocopherol mit einem Halogen-Phosphorylierungsmittel umsetzt, das erhaltene Produkt mit Ascorbinsäure, bei der die Hydroxygruppen in 5- und 6-Position mit Schutzgruppen geschützt sind, umsetzt und die Schutzgruppen entfernt.

8. Verfahren nach Anspruch 7, wobei das Halogen-Phosphorylierungsmittel Phosophoroxytrichlorid oder -oxytribromid ist und die Schutzgruppe eine Isopropylidengruppe ist.

9. Verfahren nach Anspruch 7, wobei die Schutzgruppen mittels Hydrolyse mit Salzsäure entfernt werden.

10. Pharmazeutisches Mittel, das einen Phosphorsäurediester oder ein pharmazeutisch verträgliches Salz davon nach einem der Ansprüche 1 bis 6, und einen Träger enthält.

11. Kosmetisches Mittel, das einen Phosphorsäurediester oder eine kosmetisch annehmbares Salz davon nach einem der Ansprüche 1 bis 6, und einen Träger enthält.

12. Phosphorsäurediester oder ein pharmazeutisch verträgliches Salz davon nach einem der Ansprüche 1 bis 6 zur Anwendung bei der therapeutischen Behandlung des menschlichen und tierischen Körpers oder bei der Diagnose am menschlichen oder tierischen Körper.

13. Phosphorsäurediester oder ein pharmazeutisch verträgliches Salz davon nach einem der Ansprüche 1 bis 6 zur Anwendung bei der Verabreichung von Vitaminen C und E an Mensch oder Tier.

**Revendications**

1. Diester d'acide phosphorique selon la formule:

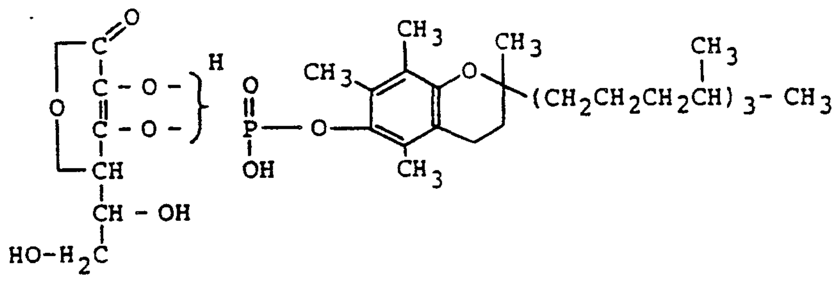

ou un sel de celui-ci.

2. Diester d'acide phosphorique ou sel de celui-ci selon la revendication 1, dans lequel le reste α-tocophérol est un reste DL-α-tocopherol.

3. Diester d'acide phosphorique ou sel de celui-ci selon la revendication 1, dans lequel le reste α-tocophérol est un reste D-α-tocopherol.

4. Sel de diester d'acide phosphorique selon l'une des revendications précédentes, dans lequel le sel est un sel de potassium.

5. Sel de diester d'acide phosphorique selon l'une des revendications 1 à 3, dans lequel le sel est un sel de sodium.

6. Sel de diester d'acide phosphorique selon l'une des revendications 1 à 3, dans lequel le sel est un sel de calcium.

7. Procédé de fabrication d'un diester d'acide phosphorique ou d'un sel de celui-ci selon l'une des revendications précédentes, qui comprend la réaction de 1'α-tocophérol avec un agent halogéno-phosphorylant, la réaction du produit réactionnel avec de l'acide ascorbique ayant des groupes hydroxyles dans les positions 5 et 6 protégés par des groupes de protection, et l'enlèvement desdits groupes de protection.

8. Procédé selon la revendication 7, dans lequel l'agent halogéno-phosphorylant est de l'oxytrichlorure de phosphore ou de l'oxytribromure de phosphore et le groupe de protection est un groupe isopropylidène.

9. Procédé selon la revendication 7, dans lequel les groupes de protection sont enlevés par hydrolyse à l'acide chlorhydrique.

10. Composition pharmaceutiquement comprenant un diester d'acide phosphorique ou un des ses sels pharmaceutiquement acceptable selon l'une des revendications 1 à 6, et un véhicule.

11. Composition cosmétique comprenant un diester d'acide phosphorique ou un de ses sels acceptable en cosmétique selon l'un des revendications 1 à 6 et un véhicule.

12. Diester d'acide phoshorique ou un de ses sels pharmaceutiquement acceptables selon l'une des revendications 1 à 6, destiné au traitement thérapeutique du corps humain ou animal ou au diagnostique pratiqué sur le corps humain ou animal.

13. Diester d'acide phosphorique ou un de ses sel pharmaceutiquement acceptables selon l'une des revendications 1 à 6, destiné à l'administration de vitamines C et E au corps humain ou animal.

FIG.1

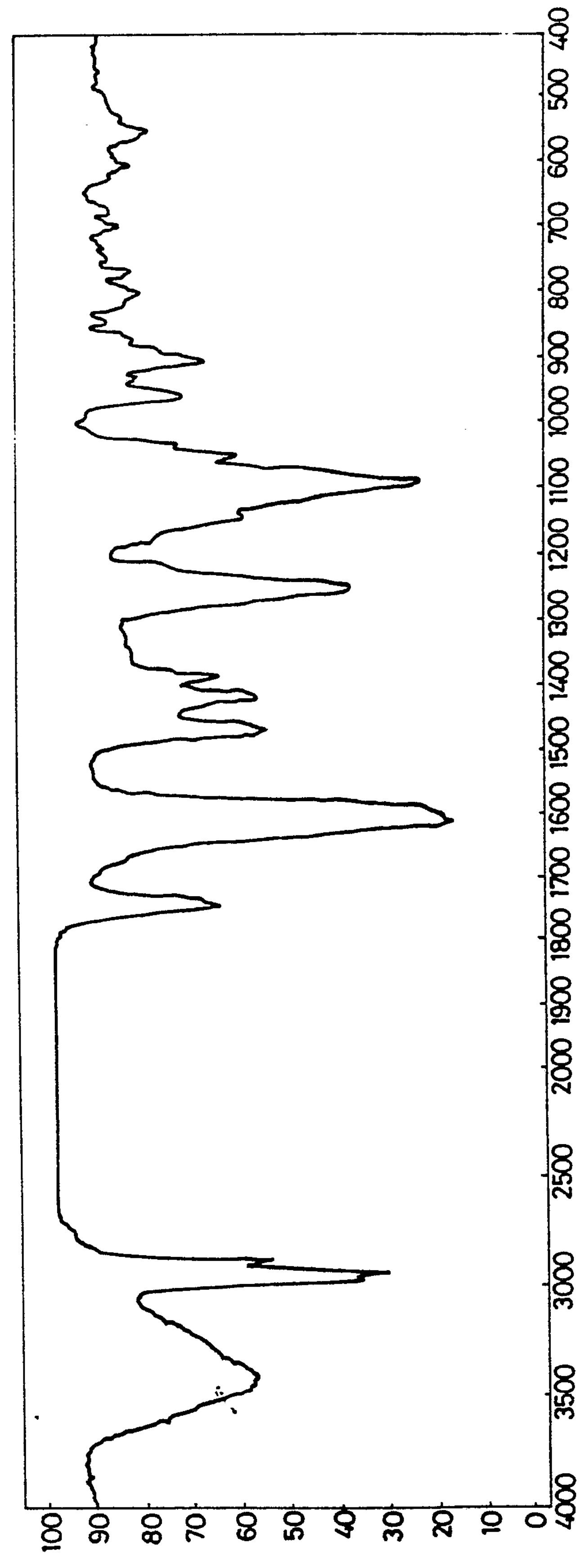
100
90
80
70
60
50
40
30
20
10
0
4000
3500
3000
2500
2000
1900
1800
1700
1600
1500
1400
1300
1200
1100
1000
900
800
700
600
500
400

FIG.2

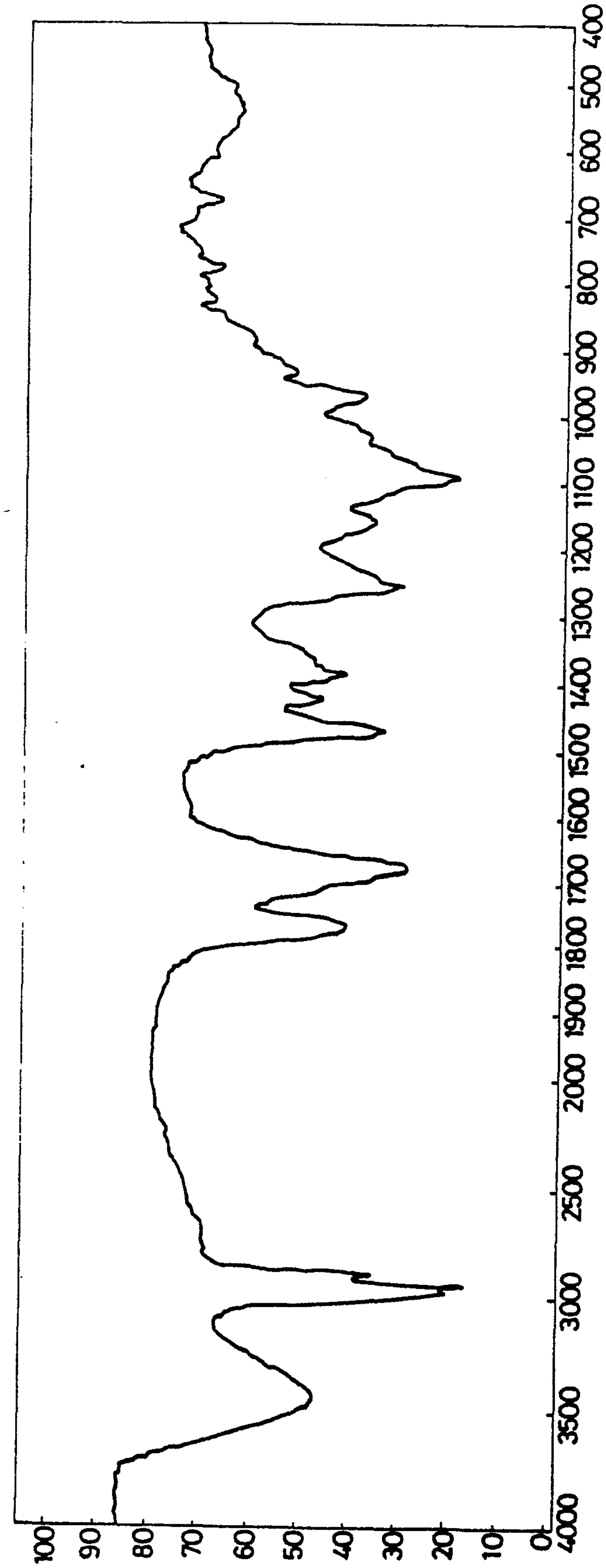
100
90
80
70
60
50
40
30
20
10
0
4000
3500
3000
2500
2000
1900
1800
1700
1600
1500
1400
1300
1200
1100
1000
900
800
700
600
500
400